# EUROPÄISCHE PATENTSCHRIFT

(11) **EP 1 893 660 B1**
(45) Veröffentlichungstag und Bekanntmachung des Hinweises auf die Patenterteilung: **03.03.2010**
(21) Anmeldenummer: 06777305.1
(22) Anmeldetag: 12.06.2006
(51) Int. Cl.: C08G 2/08, C07C 41/56

(54) **VERFAHREN ZUR HERSTELLUNG VON POLYOXYMETHYLENDIMETHYLETHERN AUS METHANOL UND FORMALDEHYD**
METHOD FOR PRODUCING POLYOXYMETHYLENE DIMETHYL ETHERS FROM METHANOL AND FORMALDEHYDE
PROCEDE DE PRODUCTION DE POLYOXYMETHYLENE-DIMETHYLETHERS A BASE DE METHANOL ET DE FORMALDEHYDE

(30) Priorität: 15.06.2005 DE 102005027701
(43) Veröffentlichungstag der Anmeldung: 05.03.2008
(73) Patentinhaber: BASF SE, 67056 Ludwigshafen (DE)
(72) Erfinder: STRÖFER, Eckhard, 68163 Mannheim (DE); HASSE, Hans, 67661 Kaiserslautern (DE); BLAGOV, Sergej, 70195 Stuttgart (DE)
(86) Internationale Anmeldenummer: PCT/EP2006/063095
(87) Internationale Veröffentlichungsnummer: WO 2006/134088

(56) Entgegenhaltungen:
- US-B1- 6 392 102

## Beschreibung

Die Erfindung betrifft ein Verfahren zur Herstellung von Polyoxymethylendimethylethern.

Polyoxymethylendimethylether stellen eine homologe Reihe der allgemeinen Formel

CH₃O(CH₂O)ₙCH₃

dar, worin n eine Zahl ≥ 1 bedeutet. Wie das Stamm-Molekül der homologen Reihe Methylal CH₃O(CH₂O)CH₃ (n = 1) sind die Polyoxymethylendimethylether Acetale. Sie werden durch Reaktion von Methanol mit wässrigem Formaldehyd in Gegenwart eines sauren Katalysators hergestellt. Wie andere Acetale sind sie unter neutralen oder alkalischen Bedingungen stabil, werden aber schon von verdünnten Säuren angegriffen. Durch Hydrolyse werden sie dabei in einem ersten Schritt zu Halbacetalen und Methanol umgesetzt. In einem zweiten Schritt werden die Halbacetale zu Formaldehyd und Methanol hydrolysiert.

Im Labormaßstab werden Polyoxymethylendimethylether durch Erhitzen von Polyoxymethylenglykol oder Paraformaldehyd mit Methanol in Gegenwart von Spuren von Schwefelsäure oder Salzsäure bei Temperaturen von 150 bis 180 °C und Reaktionszeiten von 12 bis 15 Stunden hergestellt. Dabei kommt es zu Zersetzungsreaktionen unter Bildung von Kohlendioxid und zur Bildung von Dimethylether. Bei einem Paraformaldehyd oder Polyoxymethylenglykol : Methanol-Verhältnis von 6 : 1 werden Polymere mit n > 100, im Allgemeinen n = 300 - 500 erhalten. Die Produkte werden mit Natriumsulfitlösung gewaschen und anschließend durch fraktionierte Kristallisation fraktioniert.

US 2,449,469 beschreibt ein Verfahren, bei dem Methylal mit Paraformaldehyd oder einer konzentrierten Formaldehyd-Lösung in Gegenwart von Schwefelsäure erhitzt wird. Dabei werden Polyoxymethylendimethylether mit 2 bis 4 Formaldehyd-Einheiten pro Molekül erhalten.

In jüngerer Zeit haben Polyoxymethylendimethylether als Dieselkraftstoff-Additive Bedeutung erlangt. Zur Verringerung der Rauch- und Rußbildung bei der Verbrennung von herkömmlichem Dieselkraftstoff werden diesem sauerstoffhaltige Verbindungen, welche nur wenige oder überhaupt keine C-C-Bindungen aufweisen, wie beispielsweise Methanol, zugesetzt. Jedoch sind derartige Verbindungen häufig unlöslich in Dieselkraftstoff und setzen die Cetan-Zahl und/oder den Flammpunkt des Dieselkraftstoffgemischs herab.

US 5,746,785 beschreibt die Herstellung von Polyoxymethylendimethylethern mit einem Molgewicht von 80 bis 350, entsprechend n = 1 - 10, durch Reaktion von 1 Teil Methylal mit 5 Teilen Paraformaldehyd in Gegenwart von 0,1 Gew.-% Ameisensäure bei einer Temperatur von 150 bis 240 °C beziehungsweise durch Reaktion von 1 Teil Methanol mit 3 Teilen Paraformaldehyd bei einer Temperatur von 150 bis 240 °C. Die erhaltenen Polyoxymethylendimethylether werden in Mengen von 5 bis 30 Gew.-% einem Dieselkraftstoff zugesetzt.

EP-A 1 070 755 offenbart die Herstellung von Polyoxymethylendimethylethern mit 2 bis 6 Formaldehyd-Einheiten im Molekül durch Umsetzung von Methylal mit Paraformaldehyd in Gegenwart von Trifluorsulfonsäure. Dabei werden mit einer Selektivität von 94,8 % Polyoxymethylendimethylether mit n = 2 - 5 gebildet, wobei zu 49,6 % das Dimer (n = 2) erhalten wird. Die erhaltenen Polyoxymethylendimethylether werden in Mengen von 4 bis 11 Gew.-% einem Dieselkraftstoff zugesetzt.

US 6,392,102 beschreibt die Herstellung von Polyoxymethylendimethylethern durch Umsetzung eines Einsatzstroms enthaltend Methanol und Formaldehyd, welcher durch Oxidation von Dimethylether erhalten wurde, in Gegenwart eines sauren Katalysators und gleichzeitiger Auftrennung der Reaktionsprodukte in einer katalytischen Destillationskolonne. Dabei werden Methylal, Methanol, Wasser und Polyoxymethylendimethylether erhalten.

Nachteilig an den bekannten Verfahren zur Herstellung der niederen Polyoxymethylenglykoldimethylether (mit n = 1-10) ist, dass ganz überwiegend das Dimer erhalten wird. Das als Hauptprodukt gebildete Dimer weist einen niedrigen Siedepunkt auf und setzt damit den Flammpunkt herab, wodurch es als Dieselkraftstoff-Additiv weniger geeignet ist. Oligomere mit n > 8 neigen bei niedrigen Temperaturen zur Kristallisation und sind als Dieselkraftstoff-Additive ungeeignet. Gut geeignet sind hingegen die niederen Polyoxymethylendimethylether mit n=3 und 4 (Trioxymethylenglykol- bzw. Tetraoxymethylenglykoldimethylether). Diese weisen einem typischen Dieselkraftstoffgemisch vergleichbare Siedepunkte und Flammpunkte auf. Auch wird der "cold filter plugging point" nicht heraufgesetzt.

Nachteilig an den Verfahren, die von Formaldehyd und Methanol ausgehen, ist, dass als Reaktionsprodukt Wasser gebildet wird, welches in Gegenwart der anwesenden sauren Katalysatoren bereits gebildete Polyoxymethylendimethylether hydrolysiert. Dabei werden instabile Halbacetale gebildet. Die instabilen Halbacetale setzen den Flammpunkt des Dieselkraftstoffgemischs herab und beeinträchtigen somit dessen Qualität. Ein zu niedriger Flammpunkt des Dieselkraftstoffgemischs führt aber dazu, dass die durch einschlägige DIN-Normen vorgegebenen Spezifikationen nicht mehr erfüllt werden. Halbacetale sind aber wegen vergleichbarer Siedepunkte schwer von Polyoxymethylenglykoldimethylethern abzutrennen.

Die vorstehend beschriebenen Probleme können umgangen werden, indem weitgehend wasserfrei gearbeitet wird. Dies wird erreicht durch den Einsatz von Trioxan als formaldehydhaltige Komponente, welche mit Methylal oder Dimethylether umgesetzt wird. Nun ist der Einsatzstoff Trioxan jedoch teurer als Formaldehyd, da die Trioxanherstellung ihrerseits von Formaldehyd als Einsatzstoff ausgeht. Es wird also ein zusätzlicher Verfahrensschritt benötigt.

In dem in US 6,392,102 beschriebenen Verfahren wird die Formaldehydherstellung in die Polyoxymethylendimethylether-Synthese integriert. Dabei wird Formaldehyd nicht durch oxidative Dehydrierung von Methanol - wobei im Allgemeinen wässrige Formaldehyd-Lösungen mit einem Formaldehydgehalt von 20 bis 60 Gew.-% erhalten wird - hergestellt, sondern durch oxidative Dehydrierung von Dimethylether. Dabei werden Formaldehyd-Konzentrationen von > 60 Gew.-% erreicht. Nachteilig ist die Komplexität des Gesamtverfahrens. Dieses umfasst Reaktivdestillationen, mehrere heterogenkatalytische Reaktoren, Destillationskolonnen, Absorptionskolonnen und einen Sprühturm. Dies erfordert hohe Entwicklungs- und Investitionskosten sowie Wartungskosten im laufenden Betrieb.

Es besteht also nach wie vor ein Bedarf an Verfahren zur Herstellung von Polyoxymethylenglykoldimethylethern, welche von handelsüblicher und in großen Mengen leicht verfügbarer wässriger Formaldehydlösung ausgeht. Vor dem Hintergrund ihrer Bedeutung als Dieselkraftstoffadditive besteht insbesondere ein Bedarf an der selektiven und wirtschaftlichen Herstellung von Tri- und Tetraoxymethylenglykoldimethylether.

Aufgabe der Erfindung ist es, ein verbessertes Verfahren zur selektiven Herstellung von Tri- und Tetraoxymethylenglykoldimethylether bereitzustellen, welches von wässriger Formaldehydlösung ausgeht.

Gelöst wird die Aufgabe durch ein Verfahren zur Herstellung von Tri- und Tetraoxymethylenglykoldimethylether (POMDME_{n=3,4}) durch Umsetzung von Formaldehyd mit Methanol und anschließender destillativer Aufarbeitung des Reaktionsgemischs mit den Schritten:
a) Einspeisung von wässriger Formaldehydlösung und von Methanol in einen Reaktor und Umsetzung zu einem Gemisch a enthaltend Formaldehyd, Wasser, Methylenglykol (MG), Polyoxymethylenglykole (MG_{n>1}), Methanol, Hemiformale (HF), Methylal (POMDMEₙ₌₁) und Polyoxymethylenglykoldimethylether (POMD-ME_{n>1});
b) Einspeisung des Reaktionsgemischs a in einen Reaktiwerdampfer und Auftrennung in eine Leichtsiederfraktion b1 enthaltend Formaldehyd, Wasser, Methanol, Methylenglykol, Polyoxymethylenglykole, Hemiformale, Methylal und Polyoxymethylenglykoldimethylether (POMDME_{n>1}) und eine Schwersiederfraktion b2 enthaltend Polyoxymethylenglykole, schwersiedende Hemiformale (HF_{n>1}) und schwersiedende Polyoxymethylenglykoldimethylether (POMDME_{n>4}) und Rückführung der Schwersiederfraktion b2 in den Reaktor (Schritt a));
c) Einspeisung der Leichtsiederfraktion b1 in eine erste Destillationskolonne und Auftrennung in eine Leichtsiederfraktion c1 enthaltend Formaldehyd, Wasser, Methylenglykol, Methanol, Hemiformale, Methylal, Di-, Tri- und Tetraoxymethylenglykoldimethylether (POMDME_{n=2,3,4}) und eine Schwersiederfraktion c2 enthaltend Polyoxymethylenglykole, schwersiedende Hemiformale (HF_{n>1}) und schwersiedende Polyoxymethylenglykoldimethylether (POMDME_{n>4}) und Rückführung der Schwersiederfraktion c2 in den Reaktivverdampfer (Schritt b));
d) Einspeisung der Leichtsiederfraktion c1 in eine zweite Destillationskolonne und Auftrennung in eine Leichtsiederfraktion d1 enthaltend Formaldehyd, Wasser, Methanol, Polyoxymethylenglykole, Hemiformale, Methylal und Dioxymethylenglykoldimethylether (POMDMEₙ₌₂) und eine Schwersiederfraktion d2 im Wesentlichen bestehend aus Formaldehyd, Wasser, Methylenglykol, Polyoxymethylenglykolen, Tri- und Tetraoxymethylenglykoldimethylether (POMDMEₙ₌₃,₄);
e) Einspeisung der Schwersiederfraktion d2 in einen Phasentrennapparat und Auftrennung in eine wässrige Phase e1 im Wesentlichen bestehend aus Formaldehyd, Wasser, Methylenglykol und Polyoxymethylenglykolen und eine organische Phase e2 enthaltend Tri- und Tetraoxymethylenglykoldimethylether (POMD-MEₙ₌₃,₄);
f) Einspeisung der organischen Phase e2 in eine dritte Destillationskolonne und Auftrennung in eine Leichtsiederfraktion f1 im Wesentlichen bestehend aus Formaldehyd, Wasser, Methylenglykol und Polyoxymethylenglykolen und eine Schwersiederfraktion f2 im Wesentlichen bestehend aus Tri- und Tetraoxymethylenglykoldimethylether (POMDME_{n=3,4});
g) optional Einspeisung der wässrigen Phase e1 in eine vierte Destillationskolonne und Auftrennung in eine Leichtsiederfraktion g1 im Wesentlichen bestehend aus Formaldehyd, Wasser, Methylenglykol und Polyoxymethylenglykolen und eine Schwersiederfraktion im Wesentlichen bestehend aus Wasser.

Die destillative Auftrennung des den Reaktor verlassenden, Formaldehyd, Wasser, Methylenglykol, Polyoxymethylenglykole, Methanol, Hemiformale, Methylal und Polyoxymethylenglykoldimethylether enthaltenden Gemischs ist wegen der großen Zahl der Komponenten und der großen Zahl der gleichzeitig ablaufenden chemischen Gleichgewichtsreaktionen für den Fachmann nicht offensichtlich und ein in hohem Maße anspruchsvolles Problem. Allein aus den Siedetemperaturen der enthaltenen Komponenten lassen sich für formaldehydhaltige Gemische nur wenige Aussagen bezüglich ihrer möglichen Auftrennung machen. Grund hierfür sind chemische Gleichgewichtsreaktionen, die in Gegenwart von Wasser und Methanol ablaufen und unter anderem zu Polyoxymethylenglykolen und Hemiformalen führen. Diese Reaktionen unterliegen einerseits der Limitierung durch das chemische Gleichgewicht und andererseits der kinetischen Kontrolle. Weiterhin werden reaktive Azeotrope gebildet, die zu komplexen Phasengleichgewichten führen.

In einem Schritt a) werden wässrige Formaldehydlösung und Methanol in einen Reaktor eingespeist und zu einem Gemisch a enthaltend Formaldehyd, Wasser, Methylenglykol, Polyoxymethylenglykole, Methanol, Hemiformale, Methylal und Polyoxymethylenglykoldimethylether umgesetzt.

In Schritt a) kann handelübliche wässrige Formaldehydlösung direkt eingesetzt werden oder diese kann zuvor aufkonzentriert werden, beispielsweise wie in EP-A 1 063 221 beschrieben. Im Allgemeinen beträgt die Formaldehydkonzentration der in dem erfindungsgemäßen Verfahren eingesetzten wässrigen Formaldehydlösung von 20 bis 60 Gew.-%. Methanol wird vorzugsweise in reiner Form eingesetzt. Die Gegenwart von geringen Mengen anderer Alkohole wie Ethanol ist nicht störend. Möglich ist die Verwendung von Methanol, das bis zu 30 Gew.-% Ethanol enthält.

Wasser, monomerer (freier) Formaldehyd, Methylenglykol (MG) und oligomere Polyoxymethylenglykole unterschiedlicher Kettenlänge (MG_{n>1}) liegen in wässrigen Lösungen nebeneinander in einem thermodynamischen Gleichgewicht vor, das durch eine bestimmte Verteilung der Polyoxymethylenglykole unterschiedlicher Länge gekennzeichnet ist. Der Begriff "wässrige Formaldehydlösung" bezieht sich dabei auch auf Formaldehydlösungen, die praktisch kein freies Wasser, sondern im Wesentlichen nur noch in Form von Methylenglykol bzw. in den endständigen OH-Gruppen der Polyoxymethylenglykole chemisch gebundenes Wasser enthalten. Dies ist insbesondere bei konzentrierten Formaldehydlösungen der Fall. Polyoxymethylenglykole können dabei beispielsweise zwei bis neun Oxymethyleneinheiten aufweisen. In wässrigen Formaldehydlösungen können also Dioxymethylenglykol, Trioxymethylenglykol, Tetraoxymethylenglykol, Pentaoxymethylenglyköl, Hexaoxymethylenglykol, Heptaoxymethylenglykol, Octaoxymethylenglykol und Nonaoxymethylenglykol nebeneinander vorliegen. Die Verteilung ist konzentrationsabhängig. So liegt das Maximum der Verteilung in verdünnten Formaldehydlösungen bei Homologen niedriger Kettenlänge, während es in konzentrierteren Formaldehydlösungen bei Homologen höherer Kettenlänge liegt. Eine Gleichgewichtsverschiebung hin zu längerkettigen (höhermolekularen) Polyoxymethylenglykolen kann durch Wasserentzug, beispielsweise durch einfache Destillation in einem Filmverdampfer, erfolgen. Die Gleichgewichtseinstellung erfolgt dabei mit endlicher Geschwindigkeit durch die intermolekulare Kondensation von Methylenglykol und niedermolekularen Polyoxymethylenglykolen unter Wasserabspaltung zu höhermolekularen Polyoxymethylenglykolen.

Der Reaktion von Formaldehyd mit Methanol zu Polyoxymethylenglykoldimethylether erfolgt nach der Bruttoreaktionsgleichung (1):

*nCH*₂*O* + 2*CH*₃*OH* → *CH*₃ - *O* - (*CH*₂ - *O*)*ₙ* - *CH*₃ + *H*₂*O* (1)

Der dabei eingesetzte saure Katalysator kann ein homogener oder heterogener saurer Katalysator sein. Geeignete saure Katalysatoren sind Mineralsäuren wie weitgehend wasserfreie Schwefelsäure, Sulfonsäuren wie Trifluormethansulfonsäure und para-Toluolsulfonsäure, Heteropolysäuren, saure lonenaustauscherharze, Zeolithe, Aluminosilikate, Siliciumdioxid, Aluminiumoxid, Titandioxid und Zirkondioxid. Oxidische Katalysatoren können, um deren Säurestärke zu erhöhen, mit Sulfat- oder PhosphatGruppen dotiert sein, im Allgemeinen in Mengen von 0,05 bis 10 Gew.-%. Die Umsetzung kann in einem Rührkesselreaktor (CSTR) oder einem Rohrreaktor durchgeführt werden. Wird ein heterogener Katalysator eingesetzt, ist ein Festbettreaktor bevorzugt. Wird ein Katalysator-Festbett verwendet, kann das Produktgemisch anschließend mit einem Anionenaustauscherharz in Kontakt gebracht werden, um ein im Wesentlichen säurefreies Produktgemisch zu erhalten. Im weniger vorteilhaften Fall kann auch eine Reaktivdestillation eingesetzt werden.

Die Umsetzung erfolgt im Allgemeinen bei einer Temperatur von 0 bis 200 °C, bevorzugt 50 bis 150 °C, und einem Druck von 1 bis 20 bar, bevorzugt 2 bis 10 bar.

Gemäß der Bruttoreaktionsgleichung (2) werden Polyoxymethylenglykole gebildet. Gemäß Gleichung (3) werden Polyoxymethylenglykolmonomethylether (Hemiformale, HFₙ) gebildet.

*nCH*₂*O* + *H₂O* → *HO* - (*CH*₂ - *O*)*ₙ* - *H* (2)

*nCH*₂*O* + *CH*₃*OH* → *CH*₃ - *O* - (*CH*₂ - *O*)*ₙ* - *H* (3)

Die bei der Bildung der Polyoxymethylenglykole, Hemiformale und Polyoxymethylenglykoldimethylether involvierten Kondensations- bzw. Kettenaufbaureaktionen sind Gleichgewichtsreaktionen und laufen daher (je nach Lage des chemischen Gleichgewichts) als Spaltungs- bzw. Kettenabbaureaktionen auch in umgekehrter Richtung ab. Somit ist aber jeder der in dem erfindungsgemäßen Verfahren realisierten Destillationsschritte als komplexe Reaktivdestillation aufzufassen.

In einem Schritt b) wird das Reaktionsgemischs a in einen Reaktiwerdampfer eingespeist und in eine Leichtsiederfraktion b1 enthaltend Formaldehyd, Wasser, Methanol, Methylenglykol, Polyoxymethylenglykole, Hemiformale, Methylal und Polyoxymethylenglykoldimethylether (POMDME_{n>1}) und eine Schwersiederfraktion b2 enthaltend Polyoxymethylenglykole, Hemiformale (HF_{n>1}) und Polyoxymethylenglykole (POMD-ME_{n>3}) aufgetrennt. Die Schwersiederfraktion b2 wird in den Reaktor (Schritt a) zurückgeführt.

Der Reaktiwerdampfer stellt den Sumpfverdampfer der ersten Destillationskolonne dar. Die aus der ersten Destillationskolonne zurücklaufende Fraktion c2 enthält Polyoxymethylenglykole, schwersiedende Hemiformale (HF_{n>1}) und schwersiedende Polyoxymethylenglykole (POMDME_{n>4}). Diese Fraktion vermischt sich in dem Reaktivverdampfer mit dem Reaktionsgemisch a, welches einen höheren Anteil an Wasser, Methanol, Polyoxymethylenglykolen, Hemiformalen und Polyoxymethylenglykoldimethylether kürzerer Kettenlänge enthält. So kommt es in dem Reaktiwerdampfer zur Spaltung langkettiger Komponenten in Komponenten kürzerer Kettenlänge. Der Reaktivverdampfer wird im Allgemeinen bei dem Druck der ersten Kolonne betrieben. Er kann jedoch auch bei höherem Druck betrieben werden. Der Betriebsdruck des Reaktiwerdampfers liegt im Allgemeinen bei 0,1 bis 20 bar, vorzugsweise 0,2 bis 10 bar, die Betriebstemperatur im Allgemeinen bei 50 bis 320 °C, vorzugsweise bei 80 bis 250 °C.

Die in den nachfolgend beschriebenen Schritten c), d), f) und g) eingesetzten Destillationskolonnen sind Kolonnen üblicher Bauart. In Frage kommen Füllkörperkolonnen, Bodenkolonnen und Packungskolonnen, bevorzugt sind Bodenkolonnen und Packungskolonnen. Der Begriff "Leichtsiederfraktion" wird für das im oberen Teil, der Begriff "Schwersiederfraktion" für das im unteren Teil der Kolonne entnommene Gemisch verwendet. Im Allgemeinen wird die Leichtsiederfraktion am Kolonnenkopf, die Schwersiederfraktion am Kolonnensumpf entnommen. Dies ist jedoch nicht zwingend. Möglich ist auch die Entnahme über Seitenabzüge im Abtriebs- bzw. Verstärkungsteil der Kolonne.

In einem Schritt c) wird die Leichtsiederfraktion b1 in eine erste Destillationskolonne eingespeist und in eine Leichtsiederfraktion c1 enthaltend Formaldehyd, Wasser, Methylenglykol, Methanol, Hemiformale, Methylal, Di-, Tri- und Tetraoxymethylenglykoldimethylether (POMDMEₙ₌₂,₃,₄) und eine Schwersiederfraktion c2 enthaltend Polyoxymethylenglykole, schwersiedende Hemiformale (HF_{n>1}) und schwersiedende Polyoxymethylenglykoldimethylether (POMDME_{n>4}) aufgetrennt. Die Schwersiederfraktion wird c2 in den Reaktiwerdampfer (Schritt b) zurückgeführt.

Die erste Destillationskolonne weist im Allgemeinen eine Stufenzahl von 2 bis 50, vorzugsweise von 5 bis 20 auf. Sie wird bei einem Druck von 0,1 bis 10 bar, vorzugsweise von 0,2 bis 6 bar, betrieben. Die Kopftemperatur beträgt im Allgemeinen 0 bis 260 °C, vorzugsweise 20 bis 230 °C, die Sumpftemperatur entspricht der Temperatur des Reaktivverdampfers.

In einem Schritt d) wird die Leichtsiederfraktion c1 in eine zweite Destillationskolonne eingespeist und in eine Leichtsiederfraktion d1 enthaltend Formaldehyd, Wasser, Methanol, Polyoxymethylenglykole, Hemiformale, Methylal und Dioxymethylenglykoldimethylether (POMDMEₙ₌₂) und eine Schwersiederfraktion d2 im Wesentlichen bestehend aus Formaldehyd, Wasser, Methylenglykol, Polyoxymethylenglykolen, Tri- und Tetraoxymethylenglykoldimethylether (POMDME_{n=3,4}) aufgetrennt.

"Im Wesentlichen bestehend aus" hat hier und nachfolgend die Bedeutung, dass die betreffende Fraktion zu mindestens 90 Gew.-%, vorzugsweise zu mindestens 95 Gew.-% aus den genannten Komponenten besteht. Die Schwersiederfraktion d2 enthält insbesondere praktisch keinen Dioxymethylenglykoldimethylether mehr. Dessen Gehalt in der Schwersiederfraktion d2 beträgt im Allgemeinen < 3 Gew.-%.

Die zweite Destillationskolonne weist im Allgemeinen eine Stufenzahl von 1 bis 50, vorzugsweise von 1 bis 20 auf. Sie wird bei einem Druck von 0,1 bis 10 bar, vorzugsweise von 0,2 bis 6 bar, betrieben. Die Kopftemperatur beträgt im Allgemeinen 0 bis 160 °C, vorzugsweise 20 bis 130 °C, die Sumpftemperatur beträgt im Allgemeinen 50 bis 260 °C, vorzugsweise 80 bis 220 °C.

Im Allgemeinen wird die Leichtsiederfraktion d1 in den Reaktor (Schritt a)) zurückgeführt.

In einem Schritt e) wird die Schwersiederfraktion d2 in einen Phasentrennapparat eingespeist und in eine wässrige Phase e1 im Wesentlichen bestehend aus Formaldehyd, Wasser, Methylenglykol und Polyoxymethylenglykolen und eine organische Phase e2 enthaltend Tri- und Tetraoxymethylenglykoldimethylether (POMDME_{n=3,4}) aufgetrennt.

Die organische Phase e2 enthält daneben ebenfalls noch Formaldehyd, Wasser, Methylenglykol und Polyoxymethylenglykole.

In einem Schritt f) wird die organische Phase e2 in eine dritte Destillationskolonne eingespeist und in eine Leichtsiederfraktion f1 im Wesentlichen bestehend aus Formaldehyd, Wasser, Methylenglykol und Polyoxymethylenglykolen und eine Schwersiederfraktion f2 im Wesentlichen bestehend aus Tri- und Tetraoxymethylenglykoldimethylether (POMDME_{n=3,4}) aufgetrennt.

Die dritte Destillationskolonne weist im Allgemeinen eine Stufenzahl von 1 bis 100, vorzugsweise von 1 bis 50 auf. Sie wird bei einem Druck von 0,1 bis 10 bar, vorzugsweise von 0,2 bis 6 bar, betrieben. Die Kopftemperatur beträgt im Allgemeinen 0 bis +160 °C, vorzugsweise 20 bis 130 °C, die Sumpftemperatur beträgt im Allgemeinen +100 bis +260 °C, vorzugsweise 150 bis 240 °C.

Die Schwersiederfraktion f2 stellt das Wertprodukt des erfindungsgemäßen Verfahrens dar. Sie kann mehr als 99 Gew.-% POMDME_{n=3,4} enthalten.

Im Allgemeinen wird in einem weiteren (optionalen) Schritt g) die wässrigen Phase e1 weiter aufgearbeitet. Dazu wird diese in eine vierte Destillationskolonne eingespeist und in eine Leichtsiederfraktion g1 im Wesentlichen bestehend aus Formaldehyd, Wasser, Methylenglykol und Polyoxymethylenglykolen und eine Schwersiederfraktion im Wesentlichen bestehend aus Wasser aufgetrennt.

Die vierte Destillationskolonne weist im Allgemeinen eine Stufenzahl von 1 bis 30, vorzugsweise von 1 bis 20 auf. Sie wird bei einem Druck von 0,1 bis 10 bar, vorzugsweise von 0,2 bis 6 bar, betrieben. Die Kopftemperatur beträgt im Allgemeinen -20 bis +120 °C, vorzugsweise 20 bis 100 °C, die Sumpftemperatur beträgt im Allgemeinen +40 bis +180 °C, vorzugsweise 60 bis 150 °C.

Die Leichtsiederfraktionen f1 und/oder g1 können als Rückführströme in die zweite Destillationskolonne (Schritt d)) zurückgeführt werden. Bevorzugt werden sie in die zweite Destillationskolonne zurückgeführt. Die Leichtsiederfraktionen f1 und/oder g1 können aber auch als Rückführströme in den Reaktor (Schritt a)) zurückgeführt werden.

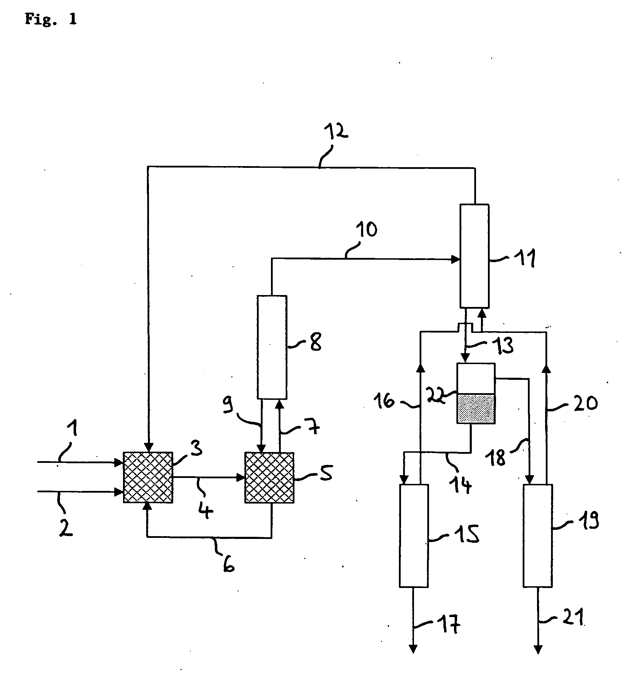

Die Erfindung wird durch die Zeichnung veranschaulicht.

Die Figur zeigt eine bevorzugte Variante des erfindungsgemäßen Verfahrens.

Wässrige Formaldehydlösung 1 und Methanol 2 werden in den Reaktor 3 eingespeist und zu einem Gemisch 4 enthaltend Formaldehyd, Wasser, Methylenglykol, Polyoxymethylenglykole, Methanol, Hemiformale, Methylal und Polyoxymethylenglykoldimethylether umgesetzt. Das Reaktionsgemisch 4 wird in dem Reaktiwerdampfer 5 in eine Leichtsiederfraktion 7 enthaltend Formaldehyd, Wasser, Methanol, Methylenglykol, Polyoxymethylenglykole, Hemiformale, Methylal und Polyoxymethylenglykoldimethylether und eine Schwersiederfraktion 6 enthaltend Polyoxymethylenglykole, schwersiedende Hemiformale und schwersiedende Polyoxymethylenglykole aufgetrennt. Die Schwersiederfraktion 6 wird in den Reaktor 3 zurückgeführt. Leichtsiederfraktion 7 wird in der ersten Destillationskolonne 8 in eine Leichtsiederfraktion 10 enthaltend Formaldehyd, Wasser, Methylenglykol, Methanol, Hemiformale, Methylal, Di-, Tri- und Tetraoxymethylenglykoldimethylether und eine Schwersiederfraktion 9 enthaltend Polyoxymethylenglykole, schwersiedende Hemiformale (HF_{n>1}) und schwersiedende Polyoxymethylenglykole aufgetrennt. Die Schwersiederfraktion 9 wird in den Reaktivverdampfer 5 zurückgeführt. Die Leichtsiederfraktion 10 wird in der zweiten Destillationskolonne in eine Leichtsiederfraktion 12 enthaltend Formaldehyd, Wasser, Methanol, Polyoxymethylenglykole, Hemiformale, Methylal und Dioxymethylenglykoldimethylether und eine Schwersiederfraktion 13 aus Formaldehyd, Wasser, Methylenglykol, Polyoxymethylenglykolen, Tri- und Tetraoxymethylenglykoldimethylether aufgetrennt. Die Leichtsiederfraktion 12 wird in den Reaktor 3 zurückgeführt. Die Schwersiederfraktion 13 wird in dem Phasentrennapparat 22 in eine wässrige Phase 18 aus Formaldehyd, Wasser, Methylenglykol und Polyoxymethylenglykolen und eine organische Phase 14 enthaltend Tri- und Tetraoxymethylenglykoldimethylether und daneben Formaldehyd, Wasser, Methylenglykol und Polyoxymethylenglykole aufgetrennt. Die organische Phase 14 wird in der vierten Destillationskolonne 15 in eine Leichtsiederfraktion 16 aus Formaldehyd, Wasser, Methylenglykol und Polyoxymethylenglykolen und eine Schwersiederfraktion 17 bestehend aus Tri- und Tetraoxymethylenglykoldimethylether aufgetrennt. Die Schwersiederfraktion 17 wird als Wertprodukt gewonnen. Die Leichtsiederfraktion 16 wird in die zweite Destillationskolonne 11 zurückgeführt. Die wässrigen Phase 18 wird in der fünfte Destillationskolonne 19 in eine Leichtsiederfraktion 20 aus Formaldehyd, Wasser, Methylenglykol und Polyoxymethylenglykolen und eine Schwersiederfraktion 21 aus Wasser aufgetrennt. Die Leichtsiederfraktion 20 wird in die zweite Destillationskolonne 11 zurückgeführt.

## Patentansprüche

1. Verfahren zur Herstellung von Tri- und Tetraoxymethylenglykoldimethylether (POMDME_{n=3,4}) durch Umsetzung von Formaldehyd mit Methanol und anschließender destillativer Aufarbeitung des Reaktionsgemischs mit den Schritten:
a) Einspeisung von wässriger Formaldehydlösung und von Methanol in einen Reaktor und Umsetzung zu einem Gemisch a enthaltend Formaldehyd, Wasser, Methylenglykol (MG), Polyoxymethylenglykole (MG_{n>1}), Methanol, Hemiformale (HF), Methylal (POMDMEₙ₌₁) und Polyoxymethylenglykoldimethylether (POMDME_{n>1});
b) Einspeisung des Reaktionsgemischs a in einen Reaktiwerdampfer und Auftrennung in eine Leichtsiederfraktion b1 enthaltend Formaldehyd, Wasser, Methanol, Methylenglykol, Polyoxymethylenglykole, Hemiformale, Methylal und Polyoxymethylenglykoldimethylether (POMDME_{n>1}) und eine Schwersiederfraktion b2 enthaltend Polyoxymethylenglykole, Hemiformale (HF_{n>1}) und Polyoxymethylenglykoldimethylether (POMDME_{n>3}) und Rückführung der Schwersiederfraktion b2 in den Reaktor (Schritt a));
c) Einspeisung der Leichtsiederfraktion b1 in eine erste Destillationskolonne und Auftrennung in eine Leichtsiederfraktion c1 enthaltend Formaldehyd, Wasser, Methylenglykol, Methanol, Hemiformale, Methylal, Di-, Tri- und Tetraoxymethylenglykoldimethylether (POMDME_{n=2,3,4}) und eine Schwersiederfraktion c2 enthaltend Polyoxymethylenglykole, schwersiedende Hemiformale (HF_{n>1}) und schwersiedende Polyoxymethylenglykoldimethylether (POMDME_{n>4}) und Rückführung der Schwersiederfraktion c2 in den Reaktiwerdampfer (Schritt b));
d) Einspeisung der Leichtsiederfraktion c1 in eine zweite Destillationskolonne und Auftrennung in eine Leichtsiederfraktion d1 enthaltend Formaldehyd, Wasser, Methanol, Polyoxymethylenglykole, Hemiformale, Methylal und Dioxymethylenglykoldimethylether (POMDMEₙ₌₂) und eine Schwersiederfraktion d2 zu mindestens 90 Gew.-% bestehend aus Formaldehyd, Wasser, Methylenglykol, Polyoxymethylenglykolen, Tri- und Tetraoxymethylenglykoldimethylether (POMDME_{n=3,4});
e) Einspeisung der Schwersiederfraktion d2 in einen Phasentrennapparat und Auftrennung in eine wässrige Phase e1 zu mindestens 90 Gew.-% bestehend aus Formaldehyd, Wasser, Methylenglykol und Polyoxymethylenglykolen und eine organische Phase e2 enthaltend Tri- und Tetraoxymethylenglykoldimethylether (POMDME_{n=3,4});
f) Einspeisung der organischen Phase e2 in eine dritte Destillationskolonne und Auftrennung in eine Leichtsiederfraktion f1 zu mindestens 90 Gew.-% bestehend aus Formaldehyd, Wasser, Methylenglykol und Polyoxymethylenglykolen und eine Schwersiederfraktion f2 zu mindestens 90 Gew.-% bestehend aus Tri- und Tetraoxymethylenglykoldimethylether (POMDME_{n=3,4});
g) optional Einspeisung der wässrigen Phase e1 in eine vierte Destillationskolonne und Auftrennung in eine Leichtsiederfraktion g1 zu mindestens 90 Gew.-% bestehend aus Formaldehyd, Wasser, Methylenglykol und Polyoxymethylenglykolen und eine Schwersiederfraktion zu mindestens 90 Gew.-% bestehend aus Wasser.

2. Verfahren nach Anspruch 1, **dadurch gekennzeichnet, dass** die Leichtsiederfraktion d1 als Rückführstrom in den Reaktor (Schritt a)) zurückgeführt wird.

3. Verfahren nach Anspruch 1 oder 2, **dadurch gekennzeichnet, dass** die Leichtsiederfraktionen f1 und/oder g1 als Rückführströme in die zweite Destillationskolonne (Schritt d)) zurückgeführt werden.

4. Verfahren nach einem der Ansprüche 1 bis 3, **dadurch gekennzeichnet, dass** die Leichtsiederfraktionen f1 und/oder g1 als Rückführströme in den Reaktor (Schritt a)) zurückgeführt werden.

## Claims

1. A process for preparing tri- and tetraoxymethylene glycol dimethyl ether (POMDME_{n=3,4}) by reacting formaldehyde with methanol and subsequently working up the reaction mixture by distillation, comprising the steps of:
a) feeding aqueous formaldehyde solution and methanol into a reactor and reacting to give a mixture a comprising formaldehyde, water, methylene glycol (MG), polyoxymethylene glycols (MG_{n>1}), methanol, hemiformals (HF), methylal (POMDMEₙ₌₁) and polyoxymethylene glycol dimethyl ethers (POMDME_{n>1});
b) feeding the reaction mixture a into a reactive evaporator and separating into a low boiler fraction b1 comprising formaldehyde, water, methanol, methylene glycol, polyoxymethylene glycols, hemiformals, methylal and polyoxymethylene glycol dimethyl ethers (POMDME_{n>1}), and a high boiler fraction b2 comprising polyoxymethylene glycols, hemiformals (HF_{n>1}) and polyoxymethylene glycol dimethyl ethers (POMDME_{n>3}), and recycling the high boiler fraction b2 into the reactor (step a));
c) feeding the low boiler fraction b1 into a first distillation column and separating into a low boiler fraction c1 comprising formaldehyde, water, methylene glycol, methanol, hemiformals, methylal, di-, tri- and tetraoxymethylene glycol dimethyl ether (POMDME_{n=2,3,4}), and a high boiler fraction c2 comprising polyoxymethylene glycols, high-boiling hemiformals (HF_{n>1}) and high-boiling polyoxymethylene glycol dimethyl ethers (POMDME_{n>4}), and recycling the high boiler fraction c2 into the reactive evaporator (step b));
d) feeding the low boiler fraction c1 into a second distillation column and separating into a low boiler fraction d1 comprising formaldehyde, water, methanol, polyoxymethylene glycols, hemiformals, methylal and dioxymethylene glycol dimethyl ether (POMDMEₙ₌₂), and a high boiler fraction d2 consisting to an extent of at least 90% by weight of formaldehyde, water, methylene glycol, polyoxymethylene glycols, tri- and tetraoxymethylene glycol dimethyl ether (POMDME_{n=3,4});
e) feeding the high boiler fraction d2 into a phase separation apparatus and separating into an aqueous phase e1 consisting to an extent of at least 90% by weight of formaldehyde, water, methylene glycol and polyoxymethylene glycols, and an organic phase e2 comprising tri- and tetraoxymethylene glycol dimethyl ether (POMDME_{n=3,4}) ;
f) feeding the organic phase e2 into a third distillation column and separating into a low boiler fraction f1 consisting to an extent of at least 90% by weight of formaldehyde, water, methylene glycol and polyoxymethylene glycols, and a high boiler fraction f2 consisting to an extent of at least 90% by weight of tri- and tetraoxymethylene glycol dimethyl ether (POMDME_{n=3,4}) ;
g) optionally feeding the aqueous phase e1 into a fourth distillation column and separating into a low boiler fraction g1 consisting to an extent of at least 90% by weight of formaldehyde, water, methylene glycol and polyoxymethylene glycols, and a high boiler fraction consisting to an extent of at least 90% by weight of water.

2. The process according to claim 1, wherein the low boiler fraction d1 is returned as a recycle stream to the reactor (step a)).

3. The process according to claim 1 or 2, wherein the low boiler fractions f1 and/or g1 are returned as recycle streams to the second distillation column (step d)).

4. The process according to any of claims 1 to 3, wherein the low boiler fractions f1 and/or g1 are returned as recycle streams to the reactor (step a)).

## Revendications

1. Procédé de fabrication d'éthers diméthyliques de tri- et tétraoxyméthylène glycol (POMDME_{n=3,4}) par réaction de formaldéhyde avec du méthanol, puis traitement distillatif du mélange réactionnel, selon les étapes :
a) introduction d'une solution aqueuse de formaldéhyde et de méthanol dans un réacteur et réaction pour former un mélange a contenant du formaldéhyde, de l'eau, du méthylène glycol (MG), des polyoxyméthylène glycols (MG_{n>1}), du méthanol, des hémiformals (HF), du méthylal (POMDMEₙ₌₁) et des éthers diméthyliques de polyoxyméthylène glycol (POMDME_{n>1}) ;
b) introduction du mélange réactionnel a dans un évaporateur réactif et séparation en une fraction de point d'ébullition bas b1 contenant du formaldéhyde, de l'eau, du méthanol, du méthylène glycol, des polyoxyméthylène glycols, des hémiformals, du méthylal et des éthers diméthyliques de polyoxyméthylène glycol (POMDME_{n>1}) et une fraction de point d'ébullition élevé b2 contenant des polyoxyméthylène glycols, des hémiformals (HF_{n>1}) et des éthers diméthyliques de polyoxyméthylène glycol (POMDME_{n>3}) et recyclage de la fraction de point d'ébullition élevé b2 dans le réacteur (étape a)) ;
c) introduction de la fraction de point d'ébullition bas b1 dans une première colonne de distillation et séparation en une fraction de point d'ébullition bas c1 contenant du formaldéhyde, de l'eau, du méthylène glycol, du méthanol, des hémiformals, du méthylal, des éthers diméthyliques de di-, tri- et tétraoxyméthylène glycol (POMDME_{n=2,3,4}) et une fraction de point d'ébullition élevé c2 contenant des polyoxyméthylène glycols, des hémiformals de point d'ébullition élevé (HF_{n>1}) et des éthers diméthyliques de polyoxyméthylène glycol de point d'ébullition élevé (POMDME_{n>4}) et recyclage de la fraction de point d'ébullition élevé c2 dans l'évaporateur réactif (étape b)) ;
d) introduction de la fraction de point d'ébullition bas c1 dans une deuxième colonne de distillation et séparation en une fraction de point d'ébullition bas d1 contenant du formaldéhyde, de l'eau, du méthanol, des polyoxyméthylène glycols, des hémiformals, du méthylal et de l'éther diméthylique de dioxyméthylène glycol (POMDMEₙ₌₂) et une fraction de point d'ébullition élevé d2 constituée à au moins 90 % en poids de formaldéhyde, d'eau, de méthylène glycol, de polyoxyméthylène glycols, d'éthers diméthyliques de tri- et tétraoxyméthylène glycol (POMDME_{n=3,4}) ;
e) introduction de la fraction de point d'ébullition élevé d2 dans un appareil de séparation de phases et séparation en une phase aqueuse e1 constituée à au moins 90 % en poids de formaldéhyde, d'eau, de méthylène glycol et de polyoxyméthylène glycols et une phase organique e2 contenant des éthers diméthyliques de tri- et tétraoxyméthylène glycol (POMDME_{n=3,4}) ;
f) introduction de la phase organique e2 dans une troisième colonne de distillation et séparation en une fraction de point d'ébullition bas f1 constituée à au moins 90 % en poids de formaldéhyde, d'eau, de méthylène glycol et de polyoxyméthylène glycols et une fraction de point d'ébullition élevé f2 constituée à au moins 90 % en poids d'éthers diméthyliques de tri- et tétraoxyméthylène glycol (POMDME_{n=3,4}) ;
g) éventuellement introduction de la phase aqueuse e1 dans une quatrième colonne de distillation et séparation en une fraction de point d'ébullition bas g1 constituée à au moins 90 % en poids de formaldéhyde, d'eau, de méthylène glycol et de polyoxyméthylène glycols et une fraction de point d'ébullition élevé constituée à au moins 90 % en poids d'eau.

2. Procédé selon la revendication 1, **caractérisé en ce que** la fraction de point d'ébullition bas d1 est recyclée en tant que courant de recyclage dans le réacteur (étape a)).

3. Procédé selon la revendication 1 ou 2, **caractérisé en ce que** les fractions de point d'ébullition bas f1 et/ou g1 sont recyclées en tant que courants de recyclage dans la deuxième colonne de distillation (étape d)).

4. Procédé selon l'une quelconque des revendications 1 à 3, **caractérisé en ce que** les fractions de point d'ébullition bas f1 et/ou g1 sont recyclées en tant que courants de recyclage dans le réacteur (étape a)).
